# EUROPEAN PATENT SPECIFICATION

(11) **EP 4 565 135 B1**
(45) Date of publication and mention of the grant of the patent: **26.11.2025**
(21) Application number: 23754537.1
(22) Date of filing: 19.07.2023
(51) Int. Cl.: A61B 5/15, A61B 5/153, A61M 5/14, A61M 25/00, A61M 39/10

(54) **HEMOLYSIS-REDUCTION EXTENSION SET FOR DIRECT BLOOD DRAW**
HÄMOLYSEREDUKTIONSVERLÄNGERUNGSSET FÜR DIREKTE BLUTENTNAHME
ENSEMBLE D'EXTENSION DE RÉDUCTION D'HÉMOLYSE POUR PRÉLÈVEMENT DE SANG DIRECT

(30) Priority: 08.09.2022 US 202263404646 P
(43) Date of publication of application: 11.06.2025
(73) Proprietor: CareFusion 303, Inc., San Diego, CA 92130 (US)
(72) Inventor: SALEHI BOROJERDI, Seyed Alireza, San Diego, CA 92130 (US); JADHAV, Amarsinh Deeliprao, Karnataka Bengaluru 560045 (IN); KHAN, Mohammed Mehtab, Karnataka Bengaluru 560045 (IN); BIERITZ, Shelby Ann, San Diego, CA 92130 (US)
(74) Representative: Zenz Patentanwälte Partnerschaft mbB
(86) International application number: PCT/US2023/028129
(87) International publication number: WO 2024/054305

(56) References cited:
- EP-A2- 0 160 807
- WO-A1-2008/048328
- WO-A1-95/21643
- US-A1- 2021 100 977
- US-A1- 2021 186 394

## Description

### TECHNICAL FIELD

The present disclosure generally relates to blood draw and administration of parenteral fluids to a patient, and particularly to systems to reduce hemolysis in PIVC blood draw using an extension set. The present invention relates to a flow restriction device according to the preamble of claim 1. Such a flow restriction device is known from US 2021/100977 A1.

### BACKGROUND

Catheters are commonly used for a variety of infusion therapies. For example, catheters may be used for infusing fluids, such as normal saline solution, various medicaments, and total parenteral nutrition, into a patient. Catheters may also be used for withdrawing blood from the patient.

A common type of catheter is an over-the-needle peripheral intravenous ("IV") catheter (PIVC). As its name implies, the over-the-needle catheter may be mounted over an introducer needle having a sharp distal tip. A catheter assembly may include a catheter hub, the catheter extending distally from the catheter hub, and the introducer needle extending through the catheter. The catheter and the introducer needle may be assembled so that the distal tip of the introducer needle extends beyond the distal tip of the catheter with the bevel of the needle facing up away from skin of the patient. The catheter and introducer needle are generally inserted at a shallow angle through the skin into vasculature of the patient.

In order to verify proper placement of the introducer needle and/or the catheter in the blood vessel, a clinician generally confirms that there is "flashback" of blood in a flashback chamber of the catheter assembly. Once placement of the needle has been confirmed, the clinician may temporarily occlude flow in the vasculature and remove the needle, leaving the catheter in place for future blood withdrawal or fluid infusion.

For blood withdrawal or collecting a blood sample from a patient, a blood collection container may be used. The blood collection container may include a syringe. Alternatively, the blood collection container may include a test tube with a rubber stopper at one end. In some instances, the test tube has had all or a portion of air removed from the test tube so pressure within the test tube is lower than ambient pressure. Such a blood collection container is often referred to as an internal vacuum or a vacuum tube. A commonly used blood collection container is a VACUTAINER^{®} blood collection tube, available from Becton Dickinson & Company.

The blood collection container may be coupled to the catheter. When the blood collection container is coupled to the catheter, a pressure in the vein is higher than a pressure in the blood collection container, which pushes blood into the blood collection container, thus filling the blood collection container with blood. A vacuum within the blood collection container decreases as the blood collection container fills, until the pressure in the blood collection container equalizes with the pressure in the vein, and the flow of blood stops.

Unfortunately, as blood is drawn into the blood collection container, red blood cells are in a high shear stress state and susceptible to hemolysis due to a high initial pressure differential between the vein and the blood collection container. Hemolysis may result in rejection and discard of a blood sample. The high initial pressure differential can also result in catheter tip collapse, vein collapse, or other complications that prevent or restrict blood from filling the blood collection container. Furthermore, blood spillage during and/or after blood draw commonly occurs.

US 2021/100977 A1 discloses devices for transferring fluid to or from a subject and include an extension tube assembly with an axially extending inner tube configured to couple to an elongate tubular cannula having opposing proximal and distal ends with an axially extending lumen and an axially extending inner tube. The inner tube extending through the tubular cannula defines an exposed needle tip and is in fluid communication with the inner tube of the extension tube assembly. The needle tip extends out of a distal end of the tubular cannula a suitable distance.

The description provided in the background section should not be assumed to be prior art merely because it is mentioned in or associated with the background section. The background section may include information that describes one or more aspects of the subject technology.

### SUMMARY

The invention is defined in the independent claim 1.

Preferable embodiments are further laid out in the dependent claims. The present disclosure provides a flow restriction device, comprising: a male luer connector portion defining a first lumen; a female luer connector portion defining a second lumen; and a flexible tube defining a third lumen, wherein the third lumen is in fluid communication with the first lumen and the second lumen, and the third lumen having a diameter about 0.508 millimeters (0.02 inches).

In some instances, the present disclosure provides a flow restriction device, comprising: a male luer connector portion defining a first lumen; a female luer connector portion defining a second lumen; and a tube defining a third lumen, wherein the third lumen is in fluid communication with the first lumen and the second lumen, and the third lumen having a diameter of about 0.508 millimeters (0.02 inches) and a length of about 33.02 millimeters (1.3 inches).

In some aspects, the present disclosure provides a peripheral intravenous catheter assembly configured to limit hemolysis during drawing of blood from a patient, comprising: a catheter hub having a proximal end and a distal end; a fluid collection device; and a flow restriction device, comprising: a male luer connector portion defining a first lumen, wherein the male luer connector portion is coupled to the catheter hub; a female luer connector portion defining a second lumen in fluid communication with the fluid collection device; and a tube defining a third lumen, wherein the third lumen is in fluid communication with the first lumen and the second lumen, and the third lumen having a diameter about 0.508 millimeters (0.02 inches).

It is understood that other configurations of the subject technology will become readily apparent to those skilled in the art from the following detailed description, wherein various configurations of the subject technology are shown and described by way of illustration. As will be realized, the subject technology is capable of other and different configurations and its several details are capable of modification in various other respects, all without departing from the scope of the subject technology. Accordingly, the drawings and detailed description are to be regarded as illustrative in nature and not as restrictive.

### BRIEF DESCRIPTION OF THE DRAWINGS

The following figures are included to illustrate certain aspects of the embodiments, and should not be viewed as exclusive embodiments. The subject matter disclosed is capable of considerable modifications, alterations, combinations, and equivalents in form and function, as will occur to those skilled in the art and having the benefit of this disclosure.
FIG. 1 illustrates a vascular access device including a peripheral intravenous catheter (PIVC) assembly that includes a flow restriction device, in accordance with some embodiments of the present disclosure.
FIG. 2 illustrates a perspective view of the flow restriction device, in accordance with some embodiments of the present disclosure.
FIG. 3 illustrates a cross-sectional view of the flow restriction device of FIG. 2, in accordance with some embodiments of the present disclosure.

### DETAILED DESCRIPTION

The detailed description set forth below describes various configurations of the subject technology and is not intended to represent the only configurations in which the subject technology may be practiced. The detailed description includes specific details for the purpose of providing a thorough understanding of the subject technology. Accordingly, dimensions may be provided in regard to certain aspects as non-limiting examples. However, it will be apparent to those skilled in the art that the subject technology may be practiced without these specific details. In some instances, well-known structures and components are shown in block diagram form in order to avoid obscuring the concepts of the subject technology.

It is to be understood that the present disclosure includes examples of the subject technology and does not limit the scope of the appended claims. Various aspects of the subject technology will now be disclosed according to particular but non-limiting examples. Various embodiments described in the present disclosure may be carried out in different ways and variations, and in accordance with a desired application or implementation.

Blood draw via a vascular access device has drawn increasing attention attributed to minimized needle sticks and improved operation efficiency as compared with traditional blood draw methods with venipuncture. Current blood draw using a peripheral intravenous catheter (PIVC) has seen some challenges, one of the most critical is hemolysis related blood quality. In particular, with currently existing PIVC products in the market, along with the standard connection (such as a short extension set and a needleless connector), and blood collection devices (such as a Vacutainer), the shear stress exerted onto blood cells tends to be on the verge of hemolyzing.

Various embodiments of the present disclosure are directed to providing systems to address hemolysis in PIVC blood draw with a hemolysis reduction accessory (also referred to herein as a flow restriction device), such as an extension set, which is pre-attached to the PIVC and serves as a flow restrictor to reduce risk of hemolysis. The hemolysis-reduction accessory is advantageously compatible with PIVC placement and does not necessitate change to any of the existing operations. The hemolysis-reduction accessory of the various embodiments described herein is potentially applicable to a wide variety of PIVC products, and compatible with existing blood collection devices and infusion disposables.

Various embodiments of the present disclosure focus on effective flow restriction with the add-on hemolysis-reduction accessory (also referred to herein as a flow restriction device) that regulates the overall flow rate of the entire fluid path as blood cells travel through. The flow restriction device can be either assembled with the PIVC or co-packaged with the PIVC. The clinician may connect a blood collection device to the port of the accessory and can then draw blood to the intended volume. After blood draw, the clinician may disconnect and discard the flow restriction device and the blood collection device together. As such, this flow restriction device can be either for single blood draw or stay inline throughout indwell.

The flow restriction devices and associated blood collection systems of the various embodiments described herein additionally provide further advantages over currently existing blood collection systems. For example, add-on flow restriction devices described herein allow for hemolysis-reduction function to be integrated for PIVC blood draw. Further, the flow restriction devices described herein are compatible with PIVC placement and allow for seamless blood draw at insertion. Additionally, since the flow restriction devices are an add-on which can be easily incorporated without any changes to existing PIVC, there is minimal impact to clinical setting and operations.

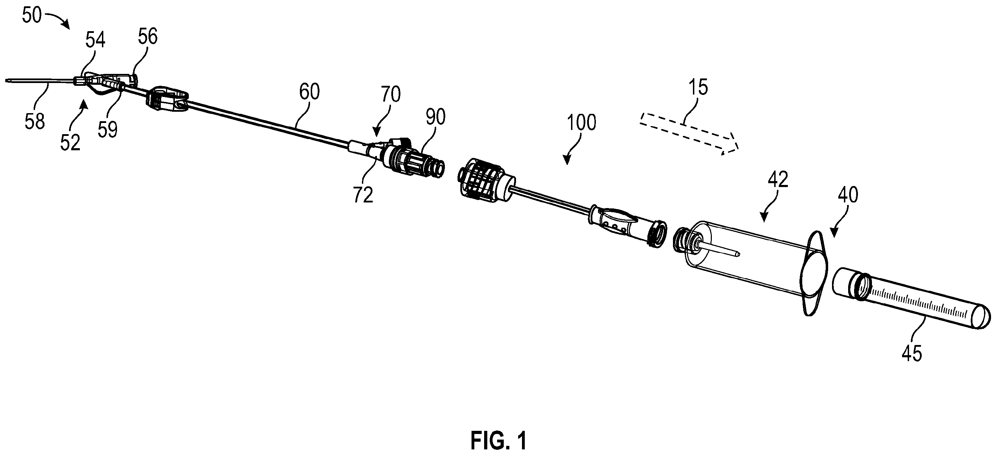

FIG. 1 illustrates a vascular access device 10 including a peripheral intravenous catheter (PIVC) assembly 50 that includes an extension set or flow restriction device 100, in accordance with some embodiments of the present disclosure. The flow restriction device 100 may be configured to extend the length of the vascular access device 10 and reduce a likelihood of hemolysis during blood collection using the vascular access device 10. In some embodiments, the vascular access device 10 may include a catheter assembly 50. The catheter assembly 50 may include a catheter hub 52, which may include a distal end 54, a proximal end 56, and a lumen extending through the distal end and the proximal end. The catheter assembly 50 may further include a catheter 58, which may be secured within the catheter hub 52 and may extend distally from the distal end 54 of the catheter hub 52. In some embodiments, the catheter assembly 50 may be a peripheral intravenous catheter (PIVC).

In some embodiments, the catheter assembly 50 may include or correspond to any suitable catheter assembly 50. In some embodiments, the catheter assembly 50 may be integrated and include an extension tube 60, which may extend from and be integrated with a side port 59 of the catheter hub 52. A non-limiting example of an integrated catheter assembly is the BD NEXIVA^{™} Closed IV Catheter system, available from Becton Dickinson and Company. In some embodiments, a proximal end of the extension tube 60 may be coupled to an adapter, such as, for example, a Y-adapter 70. In some embodiments, the flow restriction device 100 may be fluidly coupled to the Y-adapter 70.

In some embodiments, the catheter assembly 50 may be non-integrated and may not include the extension tube 60. In these and other embodiments, the flow restriction device 100 may be configured to couple to the proximal end 56 of the catheter hub 52 or another suitable portion of the catheter assembly 50. In some embodiments, the flow restriction device 100 may be coupled directly to the catheter assembly 50, eliminating the extension tube 60 and providing a compact catheter system.

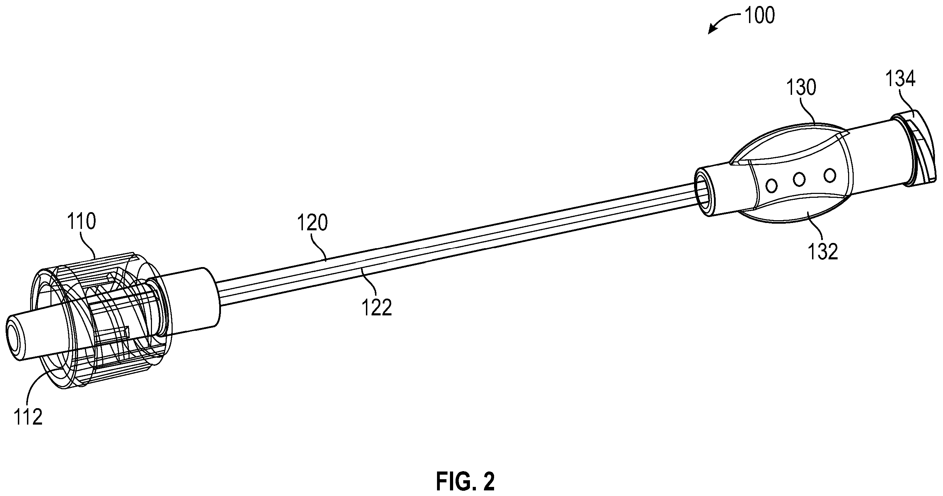

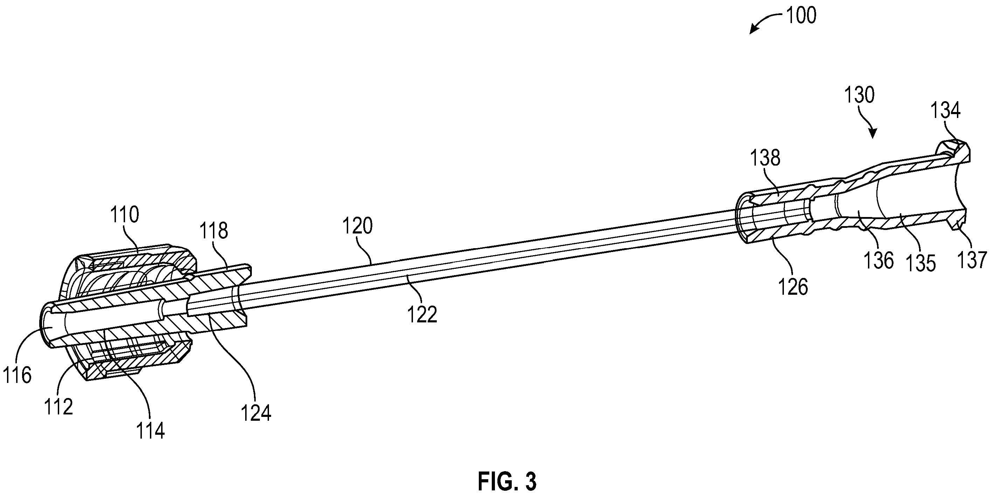

FIG. 2 illustrates a perspective view of the flow restriction device 100, in accordance with some embodiments of the present disclosure. FIG. 3 illustrates a cross-sectional view of the flow restriction device 100 of FIG. 2, in accordance with some embodiments of the present disclosure.

As illustrated in FIGS. 2 and 3, and with continued reference to FIG. 1, in some embodiments, the flow restriction device 100 may include a male luer connector portion 110 configured to couple to the catheter assembly 50. The male luer connector portion 110 may have an internal surface 112 defining a lumen 114 of the male luer connector portion 110. In some embodiments, a distal end 116 of the male luer connector portion 110 can be coupled to the catheter assembly 50.

In some embodiments, the flow restriction device 100 may further include a female luer connector portion 130 disposed proximally to the male luer connector portion 110. The female luer connector portion 130 may be configured to couple to fluid collection device 40 (e.g., a blood collection device). For example, the female luer connector portion 130 may be integrated with the blood collection device 40 or monolithically formed with the blood collection device 40 as a single unit or piece. As another example, the female luer connector portion 130 may be in the form of a female luer connector or another suitable connector, which may be coupled with a male luer portion of the blood collection device 40. The female luer connector portion 130 may include an internal surface 135 defining a lumen 136 extending therethrough for coupling to the male luer portion of the blood collection device 40. In some embodiments, a proximal end 134 of the female luer connector portion 130 can be coupled to the blood collection device 40. Optionally, a threaded portion 137 of the female luer connector portion 130 can be utilized to engage the female luer connector portion 130 to a corresponding male luer portion. In some embodiments, the female luer connector portion 130 can be rotated to engage the threaded portion 137 by apply torque to extensions 132.

The lumen 136 of the female luer connector portion 130 may be fluidly connected to the lumen 114 of the male luer connector portion 110 via a tube 120 of the flow restriction device 100, as shall be described below. In the depicted example, the flow restriction device 100 includes a tube 120 extending longitudinally therethrough and fluidly communicated with the lumens 114 and 136 of the male luer connector portion 110 and the female luer connector portion 130, respectively. In some embodiments, a distal end 124 of the tube 120 can be coupled to a proximal end 118 of the male luer connector portion 110 and a proximal end 126 of the tube 120 can be coupled to a distal end 138 of the female luer connector portion 130. Optionally, the tubing 120 can be flexible to allow portions of the vascular access device 10 to move relative to the flow restriction device 100. For example, the male luer connector portion 110 and the female luer connector portion 130 can move relative to each other.

The tube 120 may define a channel 122 along which a fluid flows from the male luer connector portion 110 into the fluid collection device 40 via the female luer connector portion 130. As depicted, the tube 120 may fluidly communicate the catheter assembly 50 with the fluid collection device 40 via the flow restriction device 100. For example, in some embodiments, a leg 72 of the Y-adapter 70 may be coupled to the flow restriction device 100. The leg 72 of Y-adapter 70 may include a lumen into which the distal end of the male luer connector portion 110 may be coupled. The Y-adapter 70 may fluidly communicate the flow restriction device 100 via a connector 90, which is depicted as a needleless connector, and the tube 120 with the catheter assembly 50, for example, via the extension tubing 60. Accordingly, the channel 122 may define a fluid pathway with a reduced, small, or micro-sized diameter (as discussed below) through which fluid entering the flow restriction device 100 from the catheter assembly 50, may flow through the flow restriction device 100 for collection in the fluid collection device 40. For example, where blood is being withdrawn or collected from a patient, the medical fluid may be blood, and the fluid collection device 40 may be a blood collection device. In some embodiments, the blood collection device may be a luer lock access device (LLAD). Accordingly, during blood collection or withdrawal from the patients, the blood sample may flow from the distal end of the male luer connector portion 110 into the LLAD 40 via the flowpath or channel 122 defined by the tube 120.

In some embodiments, channel 122 defined by the tube 120 may be an elongate, thin channel having a small, reduced, or micro-sized diameter. For example, in some embodiments, the tube 120 which defines the flowpath or micro-channel along which fluid may flow from the male luer connector portion 110 into the fluid collection device 40, may have a diameter in the range of a twenty thousandth to twenty-five thousandth of an inch. However, the various embodiments of the present disclosure are not limited to the aforementioned configuration. In some embodiments, the length of the channel 122 may range from 1 to 1.3 inches. Accordingly, during blood collection or withdrawal from the patient, the blood 15 may flow into the blood collection device 40 via the flowpath or micro-channel defined by the channel 122 having minimal diameter. The flow restriction device 100 of the various embodiments described herein is advantageous over currently existing blood collection systems. For example, during blood draw with currently existing blood draw devices, blood cells may experience wall shear stress as they flow from the distal end to the proximal end of the blood collection systems. Wall shear stress on blood cells is considered a major source of mechanical damage to blood cells causing hemolysis of the blood cells. The channel 122 having minimal diameter may facilitate increased flow resistance within the vascular access system to distribute the pressure differential and reduce shear stress experienced by the red blood cells of the blood 15. For example, the minimized diameter of the channel 122 may provide increased resistance to flow of the blood 15 and thereby decrease blood flow rate within the flow restriction device 100. Since the decreased blood flow rate causes a reduction in shear stress experienced by the red blood cells of the blood 15, a risk of hemolysis during blood collection may advantageously be reduced. Further, the geometry of the channel 122 can reduce the amount of blood wasted by currently existing blood collection systems.

Further, the flow restriction device 100 can reduce hemolysis without the use of active devices such as valves or other flow control devices. In some applications, the reduced number of parts or elements in the flow restriction device 100 can facilitate a compact design and/or high volume manufacturing.

The present disclosure is provided to enable any person skilled in the art to practice the various aspects described herein. The disclosure provides various examples of the subject technology, and the subject technology is not limited to these examples. Various modifications to these aspects will be readily apparent to those skilled in the art, and the generic principles defined herein may be applied to other aspects.

A reference to an element in the singular is not intended to mean "one and only one" unless specifically so stated, but rather "one or more." Unless specifically stated otherwise, the term "some" refers to one or more. Pronouns in the masculine (e.g., his) include the feminine and neuter gender (e.g., her and its) and vice versa. Headings and subheadings, if any, are used for convenience only and do not limit the invention.

The word "exemplary" is used herein to mean "serving as an example or illustration." Any aspect or design described herein as "exemplary" is not necessarily to be construed as preferred or advantageous over other aspects or designs. In one aspect, various alternative configurations and operations described herein may be considered to be at least equivalent.

As used herein, the phrase "at least one of" preceding a series of items, with the term "or" to separate any of the items, modifies the list as a whole, rather than each item of the list. The phrase "at least one of" does not require selection of at least one item; rather, the phrase allows a meaning that includes at least one of any one of the items, and/or at least one of any combination of the items, and/or at least one of each of the items. By way of example, the phrase "at least one of A, B, or C" may refer to: only A, only B, or only C; or any combination of A, B, and C.

A phrase such as an "aspect" does not imply that such aspect is essential to the subject technology or that such aspect applies to all configurations of the subject technology. A disclosure relating to an aspect may apply to all configurations, or one or more configurations. An aspect may provide one or more examples. A phrase such as an aspect may refer to one or more aspects and vice versa. A phrase such as an "embodiment" does not imply that such embodiment is essential to the subject technology or that such embodiment applies to all configurations of the subject technology. A disclosure relating to an embodiment may apply to all embodiments, or one or more embodiments. An embodiment may provide one or more examples. A phrase such an embodiment may refer to one or more embodiments and vice versa. A phrase such as a "configuration" does not imply that such configuration is essential to the subject technology or that such configuration applies to all configurations of the subject technology. A disclosure relating to a configuration may apply to all configurations, or one or more configurations. A configuration may provide one or more examples. A phrase such a configuration may refer to one or more configurations and vice versa.

In one aspect, unless otherwise stated, all measurements, values, ratings, positions, magnitudes, sizes, and other specifications that are set forth in this specification, including in the claims that follow, are approximate, not exact. In one aspect, they are intended to have a reasonable range that is consistent with the functions to which they relate and with what is customary in the art to which they pertain.

## Claims

1. A flow restriction device (100), comprising:
a male luer connector portion (110) defining a first lumen (114);
a female luer connector portion (130) defining a second lumen (136); and
a tube (120) defining a third lumen (122), wherein the third lumen is in fluid communication with the first lumen and the second lumen, the third lumen having a diameter of 0.508 millimeters (0.02 inches), **characterised in that** the third lumen has a length of 33.02 millimeters (1.3 inches), and the third lumen is configured to increase flow resistance through the third lumen to minimize shear stress experienced by the fluid flow.

2. The flow restriction device of Claim 1, wherein the male luer is configured to be coupled to a catheter hub (52).

3. The flow restriction device of Claim 1, wherein the female luer is configured to coupled to a fluid collection device (40).

4. The flow restriction device of Claim 1, wherein the female luer defines an extension portion (132).

5. A peripheral intravenous catheter assembly (50) configured to limit hemolysis during drawing of blood from a patient, comprising:
a catheter hub (52) having a proximal end (56) and a distal end (54);
a fluid collection device (40); and
the flow restriction device of any one of Claims 1 to 4, wherein the male luer connector portion is coupled to the catheter hub, the second lumen of the female luer connector portion is in fluid communication with the fluid collection device.

6. The peripheral intravenous catheter assembly of Claim 5, wherein the tube is flexible.

## Patentansprüche

1. Durchflussbegrenzungseinrichtung (100), aufweisend:
einen männlichen Luer-Anschlussabschnitt (110), der ein erstes Lumen (114) definiert;
einen weiblichen Luer-Anschlussabschnitt (130), der ein zweites Lumen (136) definiert; und
einen Schlauch (120), der ein drittes Lumen (122) definiert, wobei das dritte Lumen in Fluidverbindung mit dem ersten Lumen und dem zweiten Lumen steht und das dritte Lumen einen Durchmesser von 0,508 Millimetern (0,02 Zoll) aufweist, **dadurch gekennzeichnet, dass** das dritte Lumen eine Länge von 33,02 Millimetern (1,3 Zoll) aufweist und das dritte Lumen so konfiguriert ist, dass es den Strömungswiderstand durch das dritte Lumen erhöht, um die durch den Fluidstrom auftretende Scherspannung zu minimieren.

2. Durchflussbegrenzungseinrichtung nach Anspruch 1, wobei der männliche Luer so konfiguriert ist, dass er mit einer Katheternabe (52) gekoppelt werden kann.

3. Durchflussbegrenzungseinrichtung nach Anspruch 1, wobei der weibliche Luer so konfiguriert ist, dass er mit einer Fluidsammeleinrichtung (40) gekoppelt werden kann.

4. Durchflussbegrenzungseinrichtung nach Anspruch 1, wobei der weibliche Luer einen Verlängerungsabschnitt (132) definiert.

5. Periphere intravenöse Katheteranordnung (50), die so konfiguriert ist, dass sie die Hämolyse während der Blutentnahme bei einem Patienten begrenzt, aufweisend:
eine Katheternabe (52) mit einem proximalen Ende (56) und einem distalen Ende (54);
eine Fluidsammeleinrichtung (40); und
die Durchflussbegrenzungseinrichtung nach einem der Ansprüche 1 bis 4, wobei der männliche Luer-Anschlussabschnitt mit der Katheternabe gekoppelt ist und das zweite Lumen des weiblichen Luer-Anschlussabschnitts in Fluidverbindung mit der Fluidsammeleinrichtung steht.

6. Periphere intravenöse Katheteranordnung nach Anspruch 5, wobei der Schlauch flexibel ist.

## Revendications

1. Dispositif de restriction d'écoulement (100), comprenant:
une partie de connecteur Luer mâle (110) définissant une première lumière (114);
une partie de connecteur Luer femelle (130) définissant une deuxième lumière (136); et
un tube (120) définissant une troisième lumière (122), dans lequel la troisième lumière est en communication fluidique avec la première lumière et la deuxième lumière, la troisième lumière ayant un diamètre de 0,508 millimètres (0,02 pouces), **caractérisé en ce que** la troisième lumière a une longueur de 33,02 millimètres (1,3 pouces), et la troisième lumière est configurée pour augmenter la résistance à l'écoulement à travers la troisième lumière afin de minimiser la contrainte de cisaillement subie par l'écoulement de fluide.

2. Dispositif de restriction d'écoulement selon la revendication 1, dans lequel le Luer mâle est configuré pour être couplé à un embout de cathéter (52).

3. Dispositif de restriction d'écoulement selon la revendication 1, dans lequel le Luer femelle est configuré pour être couplé à un dispositif de collecte de fluide (40).

4. Dispositif de restriction d'écoulement selon la revendication 1, dans lequel le Luer femelle définit une partie d'extension (132).

5. Ensemble de cathéter intraveineux périphérique (50) configuré pour limiter l'hémolyse pendant le prélèvement de sang d'un patient, comprenant:
un embout de cathéter (52) ayant une extrémité proximale (56) et une extrémité distale (54);
un dispositif de collecte de fluide (40); et
le dispositif de restriction d'écoulement selon l'une quelconque des revendications 1 à 4, dans lequel la partie de connecteur Luer mâle est couplée à l'embout de cathéter, la deuxième lumière de la partie de connecteur Luer femelle est en communication fluidique avec le dispositif de collecte de fluide.

6. Ensemble de cathéter intraveineux périphérique selon la revendication 5, dans lequel le tube est flexible.
